# EUROPEAN PATENT APPLICATION

(11) **EP 2 704 046 A2**
(43) Date of publication of application: **05.03.2014**
(21) Application number: 13181598.7
(22) Date of filing: 23.08.2013
(51) Int. Cl.: G06F 19/00

(54) **Server device, client device, medical image processing system, and medical image porcessing method**

(30) Priority: 27.08.2012 JP 2012186667
(71) Applicant: Fujifilm Corporation, Minato-ku Tokyo (JP)
(72) Inventor: Miura, Nobuyuki, Tokyo (JP)
(74) Representative: Klunker . Schmitt-Nilson . Hirsch

(57) **Abstract**

There is provided server device processing medical image information. A storage unit stores first moving image data of a medical image and second moving image data generated by reducing a data amount of the first moving image data. A server side transmission unit transmits the second moving image data to a client device according to a request from the client device. A server side reception unit receives edition processing information transmitted from the client device and indicates the contents of edition processing with respect to the second moving image data. An edition processing unit edits the first moving image data based on the edition processing information.

## Description

### TECHNICAL FIELD

The present invention relates to a server device, a client device, a medical image processing system, a medical image processing method, and a program.

### BACKGROUND

According to the recent medical care, digital medical image data may be acquired using various modalities, such as an electronic endoscope device, a radiographic diagnosis device, and an ultrasonic diagnosis device, and diagnosis may be made based on the digital medical image data. Further, it is generally performed to treat temporal and spatial image change as an aggregate of still images called multi-frame.

In the electronic endoscope device, image data that is captured depending on release operation of the electronic endoscope is recorded as a still image or a moving image. For example, according to the electronic endoscope device described in Patent Document 1, image data that is acquired by the electronic endoscope device is temporarily stored in a medical image generation device as a moving image. Then, on the basis of an operation by an operator such as a doctor, still image data of an interest frame is extracted from the moving image data temporarily stored in the medical image generation device. The temporarily stored moving image data and the extracted still image data are to be transmitted to a server that manages medical images through a communication network.

Patent Document 1: Japanese Patent Application Publication No. 2005-103030A

According to such a medical image generation device, in the case of editing compressed moving image data that is compressed in a time axis direction, such as an interframe prediction method, recompression of the moving image data occurs, and this causes the image quality to deteriorate and causes edition processing time to be lengthened. Accordingly, it is general to perform an edition work with respect to uncompressed moving image data before performing the compression process in the time axis direction and then to perform the compression process in the time axis direction. However, the uncompressed moving image data has a large data capacity, and thus needs a great communication burden during network transmission, such as downloading.

Further, according to the medical image generation device described in Patent Document 1, the still image data is acquired by performing still image extraction processing with respect to the captured moving image. However, in the case where a client side acquires the moving image data stored in the server side and performs the still image extraction processing with respect to the moving image data, the captured moving image data is downloaded to the client side. In this case, as described above, great communication load occurs.

### SUMMARY

It is thereof an object of the present invention is to provide a server device, a client device, a medical image processing system, a medical image processing method, and a program, which can perform edition of a moving image without increasing a communication load even if moving image data has a large data capacity, and can efficiently perform edition processing of the moving image data in a short time.

The present invention can be realized by the following configurations:
(1) A server device processing medical image information, comprising:
   a storage unit storing first moving image data that is moving image data of a medical image and second moving image data that is generated by reducing a data amount of the first moving image data;
   a server side transmission unit transmitting the second moving image data to a client device according to a request from the client device;
   a server side reception unit receiving edition processing information which is transmitted from the client device and indicates the contents of edition processing with respect to the second moving image data; and
   an edition processing unit editing the first moving image data based on the received edition processing information.
(2) A client device processing medical image information, comprising:
   a client side first transmission unit transmitting transmission request information for requesting transmission of second moving image data to a server device storing first moving image data that is moving image data of a medical image and the second moving image data that is generated by reducing a data amount of the first moving image data;
   a client side reception unit receiving the second moving image data that is transmitted from the server device after transmission of the transmission request information;
   a reproduction unit reproducing the second moving image data that is received by the client side reception unit;
   an input unit inputting the contents of edition processing for the second moving image data being reproduced; and
   a client side second transmission unit transmitting edition processing information that indicates the input contents of edition processing.
(3) A medical image processing system comprising a server device processing medical image information and a client device connected to the server device through a network,
   wherein the server device includes:
   a storage unit storing first moving image data that is moving image data of a medical image and second moving image data that is generated by reducing a data amount of the first moving image data;
   a server side transmission unit transmitting the second moving image data to a client device according to a request from the client device;
   a server side reception unit receiving edition processing information which is transmitted from the client device and indicates the contents of edition processing with respect to the second moving image data; and
   an edition processing unit editing the first moving image data based on the received edition processing information, and
   wherein the client device includes:
   a client side first transmission unit transmitting transmission request information for requesting transmission of second moving image data to a server device;
   a client side reception unit receiving the second moving image data that is transmitted from the server device after transmission of the transmission request information;
   a reproduction unit reproducing the second moving image data that is received by the client side reception unit;
   an input unit inputting the contents of edition processing for the second moving image data being reproduced; and
   a client side second transmission unit transmitting the edition processing information that indicates the input contents of edition processing.
(4) A medical image processing method using a medical image processing system comprising a server device processing medical image information and a client device connected to the server device through a network, the method comprising:
   storing, in the server device, first moving image data that is moving image data of a medical image and second moving image data that is generated by reducing a data amount of the first moving image data;
   transmitting, from the client device, transmission request information for requesting transmission of second moving image data to a server device;
   receiving, by the server device, the transmission request information from the client device and transmitting the second moving image data to the client device;
   receiving, by the client device, the second moving image data that is transmitted from the server device;
   reproducing, in the client device, the received second moving image data;
   inputting the contents of edition processing for the second moving image data being reproduced;
   transmitting, from the client device, edition processing information that indicates the input contents of edition processing;
   receiving, by the server device, the edition processing information which is transmitted from the client device and indicates the contents of edition processing with respect to the second moving image data; and
   editing, in the server device, the first moving image data based on the received edition processing information.
(5) A program performing data processing in a medical image processing system comprising a server device processing medical image information and a client device connected to the server device through a network, the program realizing:
   a function of storing first moving image data that is moving image data of a medical image and second moving image data that is generated by reducing a data amount of the first moving image data;
   a function of transmitting the second moving image data to a client device according to a request from the client device;
   a function of receiving edition processing information which is transmitted from the client device and indicates the contents of edition processing with respect to the second moving image data; and
   a function of editing the first moving image data based on the received edition processing information.
(6) A program performing data processing in a medical image processing system comprising a server device processing medical image information and a client device connected to the server device through a network, the program realizing:
   a function of transmitting transmission request information for requesting transmission of second moving image data to a server device;
   a function of receiving the second moving image data that is transmitted from the server device after transmission of the transmission request information;
   a function of reproducing the second moving image data that is received by the client side reception unit;
   a function of inputting the contents of edition processing for the second moving image data being reproduced; and
   a function of transmitting the edition processing information that indicates the input contents of edition processing.

According to the present invention, the edition of the moving image is possible without increasing the communication load even if the moving image data has the large data capacity, and the edition processing of the moving image data can be efficiently performed in a short time.

### BRIEF DESCRIPTION OF THE DRAWINGS

In the accompanying drawings:
FIG. 1 is a diagram illustrating the configuration of a medical image processing system;
FIG. 2 is a block diagram illustrating the configuration of a server device 13;
FIG. 3 is a block diagram illustrating the configuration of a client device 15;
FIG. 4 is a chart diagram illustrating sequence of edition processing of endoscope moving image data;
FIG. 5 is an explanatory diagram illustrating index information of compressed moving image data; and
FIG. 6 is an explanatory diagram illustrating a state where compressed moving image data has different compression rates for respective regions.

### DETAILED DESCRIPTION

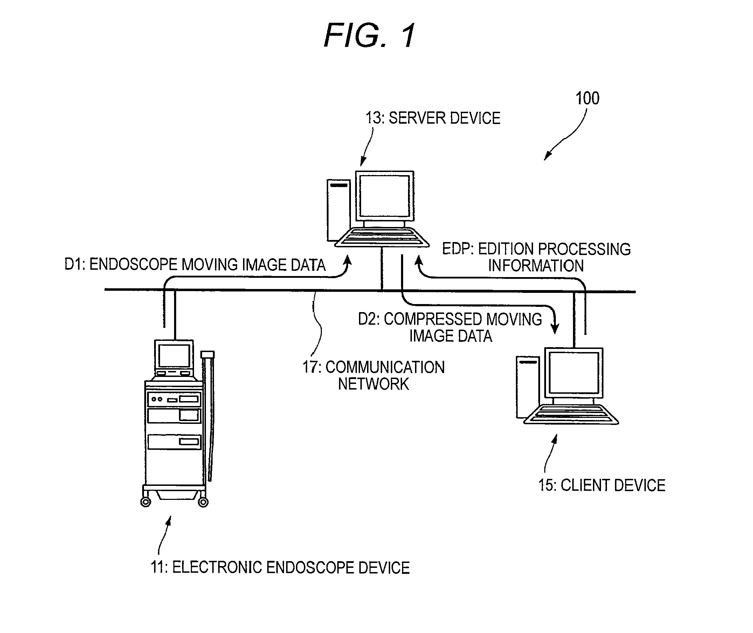

Hereinafter, preferred embodiments of the present invention will be described in detail with reference to the drawings. FIG. 1 is a diagram illustrating the configuration of a medical image processing system. A medical image processing system 100 stores endoscope moving image data D1 as medical image information that is acquired by an electronic endoscope device 11, and processes the endoscope moving image data D1 in a desired state. The medical image information is not limited to the endoscope moving image data, and may be moving image data that is acquired by various modality devices, such as, for example, a nuclear magnetic resonance device, a radiographic device, and an ultrasonic diagnosis device.

The medical image processing system 100 includes a server device 12 and a client device 15, and the server device 13, the client device 15, and the electronic endoscope device 11 that acquires the endoscope moving image data D1 are connected to each other through a communication network, such as in-hospital LAN.

The medical image processing by the medical image processing system 100 schematically becomes the following processing. The endoscope moving image data D1 acquired by the electronic endoscope device 11 is transmitted from the electronic endoscope device 11 to the server device 13, and is stored in the server device 13.

The client device 15 is used, for example, for reading of the moving image data stored in the server device 13 or making of an endoscope test report, and acquires compressed moving image data D2, which is obtained by compressing the endoscope moving image data D1, from the server device 13 based on an operation by a users such as a doctor.

The client device 15 performs edition processing with respect to the acquired compressed moving image data D2, and transmits edition processing information that indicates the contents of the edition to the server device 13.

The server device 13 processes the stored endoscope moving image data D1 based on the edition processing information EDP received from the client 15. Accordingly, the server device 13 obtains moving image data that is provided for reading or making of a report.

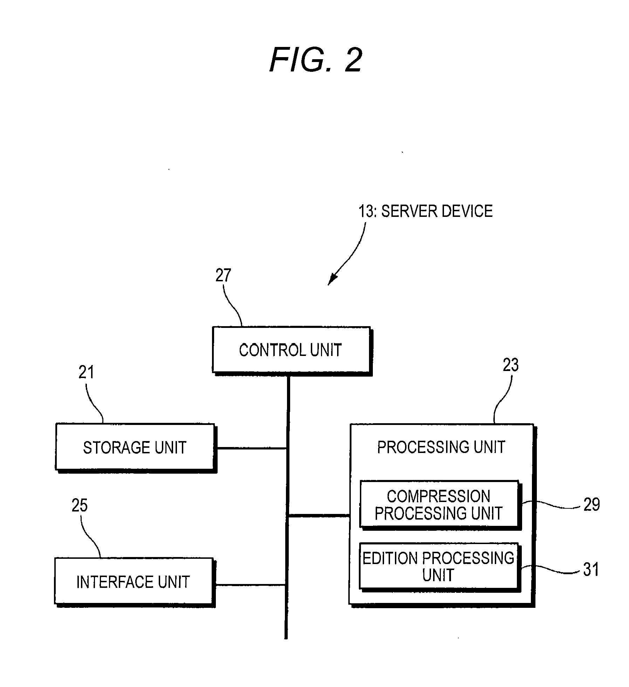

Next, the configuration of the server device 13 and the client device 15 of the medical image processing system 100 will be described. FIG. 2 is a block diagram illustrating the configuration of the server device 13. The server device 13 includes a storage unit 21 storing moving image data, a processing unit 23 performing various processes with respect to the moving image data, an interface (I/F) unit 25 connected to the communication network to perform transmission/reception of the moving image data with the electronic endoscope device 11 (see FIG. 1) or the client device 15, and a control unit 27 controlling the respective units.

The processing unit 23 includes a compression processing unit 29 compressing the endoscope moving image data (first moving image data) acquired by the electronic endoscope device 11 at a regulated or designated data reduction ratio (compression rate) and generating compressed moving image data D2 (second moving image data) of which the data amount is smaller than that of the endoscope moving image data D1, and an edition processing unit 31 performing edition processing with respect to the endoscope moving image data D1.

For the endoscope moving image data D1, for example, a non-compressed moving image data format or a moving image data format having a relatively low compression rate, such as Motion JPEG, is used. For the compressed moving image data D2, for example, a moving image data format having a relatively high compression rate, such as H.264, is used.

The storage unit 21 stores the endoscope moving image data D1 and the compressed moving image data D2 that is generated from the endoscope moving image data in association with each other.

The edition processing unit 31 edits the endoscope moving image data D1 based on the edition processing information EDP, which is transmitted through the interface unit 25, to be described later.

In this configuration example, as a transfer method of the compressed moving image data D2, a streaming method, which is a communication method that can reproduce data while receiving the data, has been used. That is, the control unit 27 also functions as a transmission unit that performs streaming distribution with respect to the compressed moving image data D2 compressed by the compression processing unit 29.

The edition processing of the endoscope moving image data D1 may be extraction processing of partial moving image data, which corresponds to a part of the endoscope moving image data D1 on the time axis, or extraction processing of still image data (one sheet of still image among the Motion JPEG moving image) that constitutes the endoscope moving image data D1. Further, the edition processing of the endoscope moving image data D1 may include image processing or compression processing that is performed with respect to the extracted image data.

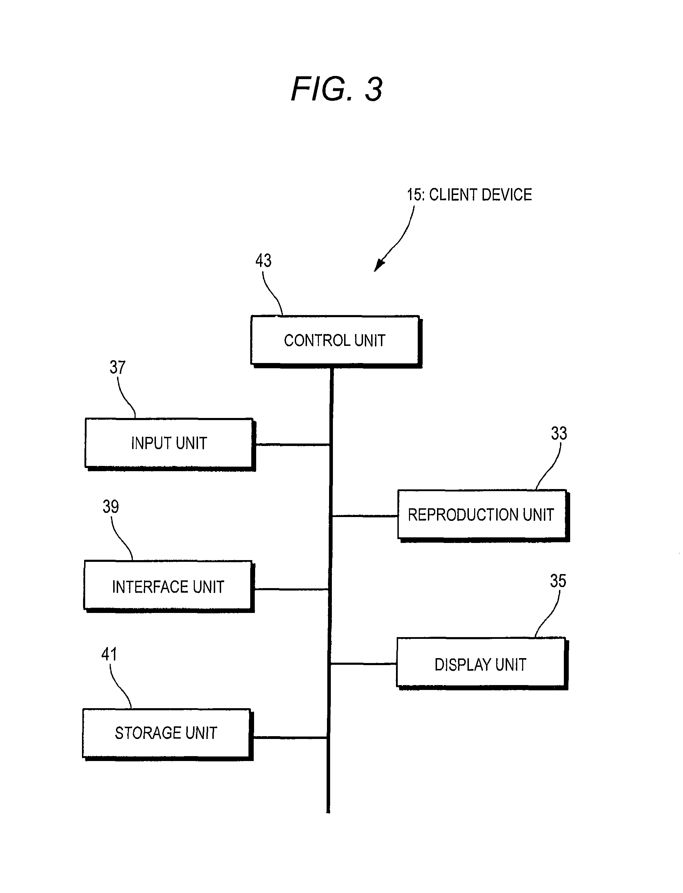

FIG. 3 is a block diagram illustrating the configuration of the client device 15. The client device 15 sets the contents of edition processing with respect to the endoscope moving image data D1. The client device 15 includes a reproduction unit 33, a display unit 35, a compression unit 37, an interface (I/F) unit 39 connected to the communication network to perform transmission/reception of the moving image data with the server device 13, a storage unit 41 storing the received moving image data, and a control unit 43 controlling the respective units. The control unit 43 also functions as a transmission unit transmitting information to the server device 13.

The reproduction unit 33 reproduces the compressed moving image data D2 that is transmitted from the server device 13. In this configuration example, the compressed moving image data D2 is transmitted from the server device 13 in the streaming method, and the reproduction unit 33 may sequentially reproduce the received data streams without waiting the reception completion of the compressed moving image data D2.

The display unit 35 includes a liquid crystal monitor that displays the reproduced moving image or various kinds of information.

The input unit 37 receives an operation by a user, such as a doctor, and inputs the contents of edition processing with respect to the reproduced compressed moving image data D2. As described above, the contents of edition processing includes extraction processing of the partial moving image data that is a part on the time axis or still image data, and may also include image processing or compression processing that is performed with respect to the extracted image data.

The work to input the contents of edition processing from the input unit 37 is performed by a user to designate positions of a start point and an end point of the extracted compressed moving image data using an input device, such as a keyboard or a mouse, while referring to the image of the compressed moving image data D2 that is displayed on the display unit 35. Further, if needed, the image processing or compression processing is designated.

In accordance with the input result of the contents of edition processing, the control unit 43 generates edition processing information that is indicated by a time code corresponding to the start point and the end point and a frame number, and edition processing information such as various kinds of image processing or compression processing.

The medical image processing system 100 having the above-described configuration generates the compressed moving image data D2 from the endoscope moving image data D1 that is captured by the electronic endoscope device 11 and stored in the server device 13, and transmits the compressed moving image data D2 to the client device 15.

Since the server device 13 does not transmit the endoscope moving image data D1 having a large data capacity to the client device 15 as it is, but transmits the compressed moving image data, of which the data capacity is reduced and the communication burden is also reduced, an operation burden of the server device 13 is reduced.

Further, the server device 13 transmits the compressed moving image data D2 in the streaming method so that the client device 15 can reproduce the moving image data while receiving the moving image data.

In the case of inputting the contents of edition processing, the client device 15 uses the compressed moving image data D2. Unlike the image diagnosis, the main purpose of the work to input the contents of edition processing is to cut off the moving image data, and thus it is not necessary for the moving image data to have a high picture quality.

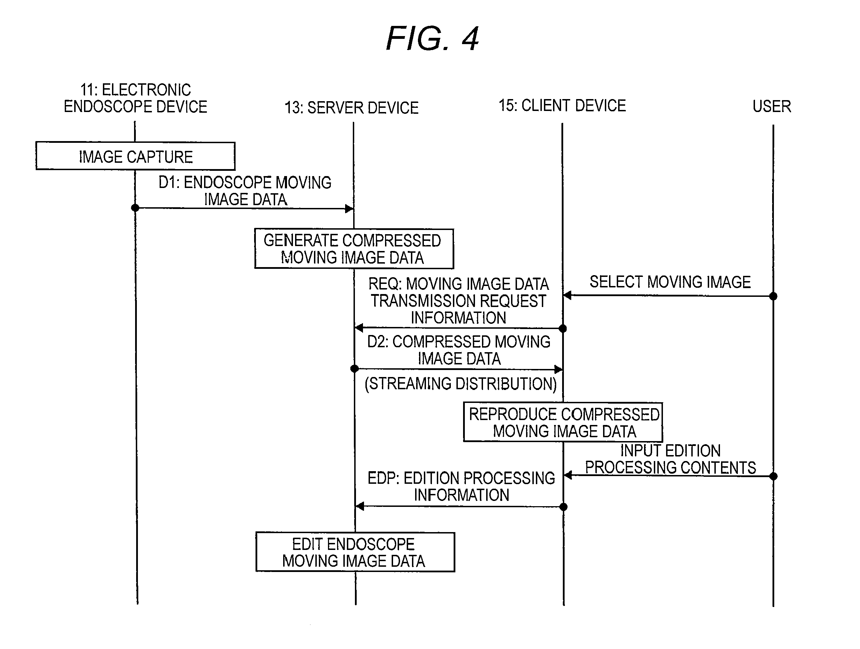

Next, the contents of edition processing with respect to the endoscope moving image data, which is performed by the medical image processing system 100, will be described in detail. FIG. 4 is a chart diagram illustrating the sequence of edition processing with respect to the endoscope moving image data.

The server device 13 receives the endoscope moving image data D1 that is acquired by the electronic endoscope device 11, and stores the endoscope moving image data D1 in the storage unit 21 (see FIG. 2) of the server device 13.

The server device 13 stores the input endoscope moving image data, generates the compressed moving image data D2 from the endoscope moving image data D1, and stores the generated compressed moving image data D2 in association with the endoscope moving image data D1.

The endoscope moving image data D1 is accumulated in the storage unit 21 at every endoscopy. Accordingly, in the storage unit 21, pairs of endoscope image data D1 and compressed moving image data D2 in association with the endoscope image data are stored.

The client device 15 displays a list of endoscope moving image data D1 stored in the server device 13 on the display unit 35 together with various kinds of information, such as a thumbnail image, data storage name, patient ID, and inspection date and time. The user selects desired moving image data among plural pieces of moving image data stored in the server device 13 as an edition target by an operation through the input unit 37.

The client device 15 sends request information REQ for requesting transmission of moving image data selected by the user to the server device 13. The request signal REQ includes identification information indicating which endoscope moving image data D1 (compressed moving image data D2) the selected moving image data is.

If the request information REQ is received from the client device 15, the server device 13 transmits the compressed moving image data, which is associated with the endoscope moving image data that corresponds to the identification information of the request information REQ, to the client device 15 that has sent the request information in the streaming method.

The client device 15 starts reception of the compressed moving image data D2 that is streaming-distributed from the server device 13. The client device 15 may reproduce the compressed moving image data D2 being received even before the streaming distribution is completed. Accordingly, the client device 15 can perform edition processing with elimination of a downloading waiting time, and thus can reproduce the received compressed moving image data D2 without keeping a user waiting.

The client device 15 displays the image of the received compressed moving image data D2 on the display unit 35. The user inputs the contents of edition processing with respect to the moving image through the input unit 37 of the client device 15 while reading the moving image being reproduced.

The client device 15 transmits the edition processing information EDP that indicates the contents of edition processing input by the user to the server device 13.

If the edition processing information EDP is received from the client device 15, the server device 13 performs the edition processing with respect to the endoscope moving image data D1 that corresponds to the compressed moving image data D2 stored in the storage unit 21 based on the edition processing information EDP.

Specifically, the edition processing unit 31 of the server device 13 performs extraction processing to cut off the partial moving image data or the still image data based on the extraction position information of the start point and the end point included in the edition processing information EDP. The extraction processing is to selectively extract one or plural pieces of partial moving image data or still image data according to the extraction position information included in the edition processing information EDP.

If other processing information, such as image processing or compression processing, is included in the edition processing information EDP in relation to the extraction position information, the edition processing unit 31 performs designated processing with respect to extracted image data that corresponds to the extraction position information. Table 1 shows an example of the contents of the edition processing information EDP.

**[Table 1]**

| Extraction Position Information | | | Processing Information | |
|---|---|---|---|---|
| | Start Position | End Position | Processing Contents | Processing Parameter |
| Region A | P1 | P2 | Image Processing A | Parameters a1, a2, ... |
| Region B | P3 | P4 | Image Processing B | Parameters b1, b2, ... |
| | | | Image Processing C | Parameters c1, c2, ... |

For example, if regions A and B are registered as the extraction position information, and as the processing information, region A corresponds to image processing A, and region B corresponds to image processing B and compression processing C, the edition processing unit 31 performs image processing A with respect to extracted image data A, and performs image processing B and compression processing C with respect to extracted image data B.

As described above, since the edition processing information EDP includes extracted image data information and processing information for the extracted image data, it is possible to adjust the picture quality of the extracted image data, for example, to a level that is appropriate for the diagnosis. Further, the processing parameters may be any parameters that are set for respective processing contents, and may be prescribed values or values input from the client device 15.

After the edition processing unit 31 performs the edition processing with respect to the endoscope moving image data D1 based on the edition processing information EDP, the server device 13 stores the moving image data after the edition processing in the storage unit 21. Accordingly, the moving image data after the edition processing is in a state where it can be taken out any time. Further, the moving image data after high-resolution edition processing can be easily read as patient information or can be easily used for making of an endoscope text report.

Since the compressed moving image data D2 having a reduced data amount and the edition processing information (EDP) indicating the contents of edition processing are transmitted and received between the server device 13 and the client device 15, instead of the endoscope moving image data D1 having large data amount, the edition processing with respect to the endoscope moving image data D1 is performed. According to this method, the load which is applied to the communication network that connects the server device 13 and the client device 15 to each other can be reduced.

Further, the above-described medical image processing system 100 may edit the endoscope moving image data as follows. In the compressed moving image data D2 stored in the server device 13, the position of an interest frame may be pre-specified through the input by the user or operation processing, such as automatic extraction processing of a feature amount, by the server device 13 during the image capturing by the electronic endoscope device 11. In this case, the server device 13 transmits information on the specified frame position to the client device 15 together with the compressed moving image data D2.

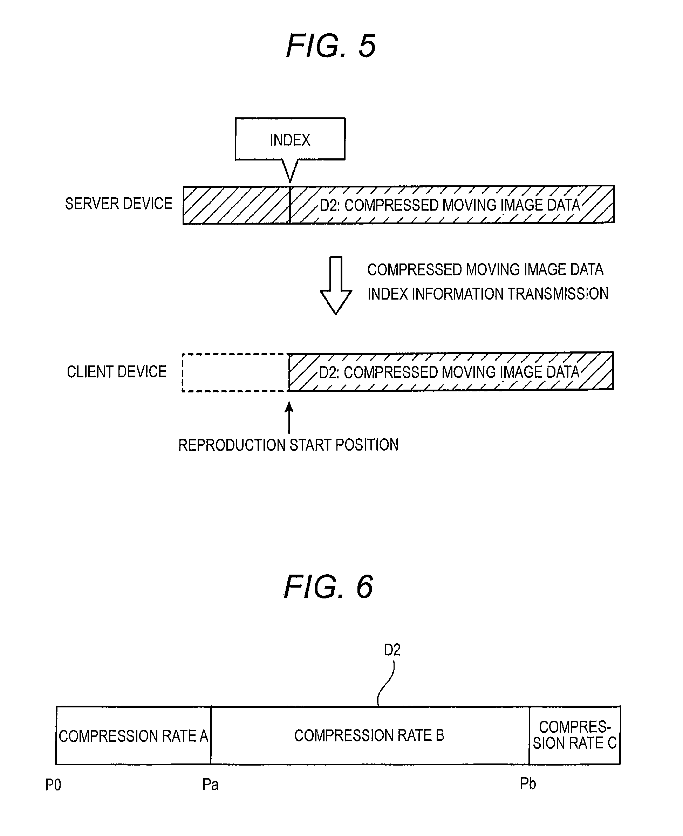

As illustrated in FIG. 5, when transmitting the compressed moving image data D2 stored in the storage unit 21 to the client device 15, the server device 13 also transmits index information that indicates the position of the interest frame in the compressed moving image data D2. The index information may be a still image at the specified frame position in addition to the position information that indicates the specified frame position.

The client device 15 controls starting of the reproduction from the specified frame position of the compressed moving image data D2 based on the received index information. If the index information is a still image, the client device 15 searches for the position where the still image appears by an image comparison operation method or the like, and starts reproduction from the position where the coincident image appears.

By transmitting the index information together with the compressed moving image data D2, reproduction of unimportant moving image is automatically omitted and the reproduction may be performed from the interest frame on the side of the client device 15. Accordingly, the user's edition processing can be efficiently performed.

Further, as illustrated in FIG. 6, the compressed moving image data D2 may not have a constant image compression rate as a whole, but may have regions with plural kinds of compression rates. For example, a low compression rate is set with respect to the moving image of an observation portion that is important to the diagnosis, and a high compression rate may be set with respect to the moving image of a relatively unimportant portion. Accordingly, even in the case of recording for a long time, the increase of the capacity of data stored in the server device 13 can be suppressed while reproduction of high-resolution moving image can be performed with respect to the important portion.

The change position of the compression rate is set by an operator through a switch that is installed on an endoscope hand function unit during the image capturing by the electronic endoscope device 11. However, a user of the client device 15 may perform such setting while viewing the compressed moving image data D2 that is displayed on the display unit 35, or various setting forms may be adopted.

In the case of setting the change position of the compression rate during image capturing, the index information that indicates the change position is included in a meta file of the endoscope moving image data, and the server device 13 reads the index information and transmits the index information to the client device 15 together with the compressed moving image data D2.

As described above about the main configuration, the server device 13 and the client device 15 include known hardware configuration, such as a CPU, a main storage device, an auxiliary storage device, an input/output interface, a communication interface, an input device (mouse and keyboard), a display device (display monitor), and a data bus, and a known operating system is installed therein. The medical image processing according tot the present invention is realized by executing the installed program from a recording medium such as CD-ROM. Further, the program may be installed after being downloaded from the storage device of the server that is connected through the network such as the Internet.

The present invention is not limited to the above-described embodiments, and combinations of respective configurations in the embodiments and configurations that can be changed or applied by those skilled in the art based on the description and known technology are planned by the present invention, and are included within the range for the protection.

As described above, the description discloses the followings.
(1) A server device processing medical image information including: a storage unit storing first moving image data that is moving image data of a medical image and second moving image data that is generated by reducing a data amount of the first moving image data; a server side transmission unit transmitting the second moving image data to a client device according to a request from the client device; a server side reception unit receiving edition processing information which is transmitted from the client device and indicates the contents of edition processing with respect to the second moving image data; and an edition processing unit editing the first moving image data based on the received edition processing information.
(2) In the server device of (1), the server side transmission unit transmits the second moving image data in a reproducible communication method while receiving the second moving image data.
(3) In the server device of (1) or (2), the contents of edition processing with respect to the second moving image data include extraction processing of partial moving image data that is a part of the second moving image data on a time axis or still image data, the edition processing information includes extraction position information that indicates an extraction range of the partial moving image data from the second moving image data or the still image data, and the edition processing unit extracts extracted image data, which corresponds to the partial moving image data or the still image data of the extraction range indicated by the extraction position information, from the first moving image data.
(4) In the server device of (3), the edition processing unit performs processing that includes at least one of image processing and compression processing with respect to the extracted image data extracted from the first moving image data.
(5) In the server device of (3) or (4), the storage unit stores index information that indicates a specified frame position of the first moving image data.
(6) In the server device of (5), the storage unit stores a still image of the specified frame position as the index information.
(7) In the server device of any one of (1) to (6), the second moving image data is configured to include moving image data of different kinds of data amount reduction rates.
(8) A client device processing medical image information including: a client side first transmission unit transmitting transmission request information for requesting transmission of second moving image data to a server device storing first moving image data that is moving image data of a medical image and the second moving image data that is generated by reducing a data amount of the first moving image data; a client side reception unit receiving the second moving image data that is transmitted from the server device after transmission of the transmission request information; a reproduction unit reproducing the second moving image data that is received by the client side reception unit; an input unit inputting the contents of edition processing for the second moving image data being reproduced; and a client side second transmission unit transmitting edition processing information that indicates the input contents of edition processing.
(9) In the client device of (8), the reproduction unit reproduces the second moving image data while receiving the second moving image data.
(10) In the client device of (8) or (9), the contents of edition processing with respect to the second moving image data are extraction processing of partial moving image data that is a part of the second moving image data on a time axis or still image data, and the edition processing information includes extraction position information that indicates an extraction range of the extracted partial moving image data or the still image data from the second moving image data.
(11) In the client device of (10), the contents of edition processing include at least at lest one of image processing and compression processing with respect to the extracted image data from the second moving image data that corresponds to the extracted partial moving image data or the still image data.
(12) In the client device of any one of (8) to (11), index information that indicates a specified frame position of the first moving image data is received from the server device, and the reproduction unit starts reproduction from the specified frame position of the second moving image data based on the index information.
(13) In the client device of (12), the client side reception unit receives a still image of the specified frame from the server device as the index information, and the reproduction unit starts reproduction from a position of the still image of the second moving image data.
(14) A medical image processing system comprising a server device processing medical image information and a client device connected to the server device through a network, wherein the server device includes: a storage unit storing first moving image data that is moving image data of a medical image and second moving image data that is generated by reducing a data amount of the first moving image data; a server side transmission unit transmitting the second moving image data to a client device according to a request from the client device; a server side reception unit receiving edition processing information which is transmitted from the client device and indicates the contents of edition processing with respect to the second moving image data; and an edition processing unit editing the first moving image data based on the received edition processing information, and the client device includes: a client side first transmission unit transmitting transmission request information for requesting transmission of second moving image data to a server device; a client side reception unit receiving the second moving image data that is transmitted from the server device after transmission of the transmission request information; a reproduction unit reproducing the second moving image data that is received by the client side reception unit; an input unit inputting the contents of edition processing for the second moving image data being reproduced; and a client side second transmission unit transmitting the edition processing information that indicates the input contents of edition processing.
(15) A medical image processing method using a medical image processing system comprising a server device processing medical image information and a client device connected to the server device through a network, includes: storing, in the server device, first moving image data that is moving image data of a medical image and second moving image data that is generated by reducing a data amount of the first moving image data; transmitting, from the client device, transmission request information for requesting transmission of second moving image data to a server device; receiving, by the server device, the transmission request information from the client device and transmitting the second moving image data to the client device; receiving, by the client device, the second moving image data that is transmitted from the server device; reproducing, in the client device, the received second moving image data; inputting the contents of edition processing for the second moving image data being reproduced; transmitting, from the client device, edition processing information that indicates the input contents of edition processing; receiving, by the server device, the edition processing information which is transmitted from the client device and indicates the contents of edition processing with respect to the second moving image data; and editing, in the server device, the first moving image data based on the received edition processing information.
(16) A program performing data processing in a medical image processing system comprising a server device processing medical image information and a client device connected to the server device through a network, realizing: a function of storing first moving image data that is moving image data of a medical image and second moving image data that is generated by reducing a data amount of the first moving image data; a function of transmitting the second moving image data to a client device according to a request from the client device; a function of receiving edition processing information which is transmitted from the client device and indicates the contents of edition processing with respect to the second moving image data; and a function of editing the first moving image data based on the received edition processing information.
(17) A program performing data processing in a medical image processing system comprising a server device processing medical image information and a client device connected to the server device through a network, realizing: a function of transmitting transmission request information for requesting transmission of second moving image data to a server device; a function of receiving the second moving image data that is transmitted from the server device after transmission of the transmission request information; a function of reproducing the second moving image data that is received by the client side reception unit; a function of inputting the contents of edition processing for the second moving image data being reproduced; and a function of transmitting the edition processing information that indicates the input contents of edition processing.

## Claims

1. A server device processing medical image information, comprising:
a storage unit storing first moving image data that is moving image data of a medical image and second moving image data that is generated by reducing a data amount of the first moving image data;
a server side transmission unit transmitting the second moving image data to a client device according to a request from the client device;
a server side reception unit receiving edition processing information which is transmitted from the client device and indicates the contents of edition processing with respect to the second moving image data; and
an edition processing unit editing the first moving image data based on the received edition processing information.

2. The server device according to claim 1, wherein the server side transmission unit transmits the second moving image data in a reproducible communication method while receiving the second moving image data.

3. The server device according to claim 1 or 2,
wherein the contents of edition processing with respect to the second moving image data include extraction processing of partial moving image data that is a part of the second moving image data on a time axis or still image data,
wherein the edition processing information includes extraction position information that indicates an extraction range of the partial moving image data from the second moving image data or the still image data, and
wherein the edition processing unit extracts extracted image data, which corresponds to the partial moving image data or the still image data of the extraction range indicated by the extraction position information, from the first moving image data.

4. The server device according to claim 3, wherein the edition processing unit performs processing that includes at least one of image processing and compression processing with respect to the extracted image data extracted from the first moving image data.

5. The server device according to claim 3 or 4, wherein the storage unit stores index information that indicates a specified frame position of the first moving image data.

6. The server device according to claim 5, wherein the storage unit stores a still image of the specified frame position as the index information.

7. The server device according to any one of claims 1 to 6, wherein the second moving image data is configured to include moving image data of different kinds of data amount reduction rates.

8. A client device processing medical image information, comprising:
a client side first transmission unit transmitting transmission request information for requesting transmission of second moving image data to a server device storing first moving image data that is moving image data of a medical image and the second moving image data that is generated by reducing a data amount of the first moving image data;
a client side reception unit receiving the second moving image data that is transmitted from the server device after transmission of the transmission request information;
a reproduction unit reproducing the second moving image data that is received by the client side reception unit;
an input unit inputting the contents of edition processing for the second moving image data being reproduced; and
a client side second transmission unit transmitting edition processing information that indicates the input contents of edition processing.

9. The client device according to claim 8, wherein the reproduction unit reproduces the second moving image data while receiving the second moving image data.

10. The client device according to claim 8 or 9,
wherein the contents of edition processing with respect to the second moving image data are extraction processing of partial moving image data that is a part of the second moving image data on a time axis or still image data, and
wherein the edition processing information includes extraction position information that indicates an extraction range of the extracted partial moving image data or the still image data from the second moving image data.

11. The client device according to claim 10, wherein the contents of edition processing include at least at lest one of image processing and compression processing with respect to the extracted image data from the second moving image data that corresponds to the extracted partial moving image data or the still image data.

12. The client device according to any one of claims 8 to 11,
wherein index information that indicates a specified frame position of the first moving image data is received from the server device, and
wherein the reproduction unit starts reproduction from the specified frame position of the second moving image data based on the index information.

13. The client server according to claim 12,
wherein the client side reception unit receives a still image of the specified frame from the server device as the index information, and
wherein the reproduction unit starts reproduction from a position of the still image of the second moving image data.

14. A medical image processing system comprising a server device processing medical image information and a client device connected to the server device through a network,
wherein the server device includes:
a storage unit storing first moving image data that is moving image data of a medical image and second moving image data that is generated by reducing a data amount of the first moving image data;
a server side transmission unit transmitting the second moving image data to a client device according to a request from the client device;
a server side reception unit receiving edition processing information which is transmitted from the client device and indicates the contents of edition processing with respect to the second moving image data; and
an edition processing unit editing the first moving image data based on the received edition processing information, and
wherein the client device includes:
a client side first transmission unit transmitting transmission request information for requesting transmission of second moving image data to a server device;
a client side reception unit receiving the second moving image data that is transmitted from the server device after transmission of the transmission request information;
a reproduction unit reproducing the second moving image data that is received by the client side reception unit;
an input unit inputting the contents of edition processing for the second moving image data being reproduced; and
a client side second transmission unit transmitting the edition processing information that indicates the input contents of edition processing.

15. A medical image processing method using a medical image processing system comprising a server device processing medical image information and a client device connected to the server device through a network, the method comprising:
storing, in the server device, first moving image data that is moving image data of a medical image and second moving image data that is generated by reducing a data amount of the first moving image data;
transmitting, from the client device, transmission request information for requesting transmission of second moving image data to a server device;
receiving, by the server device, the transmission request information from the client device and transmitting the second moving image data to the client device;
receiving, by the client device, the second moving image data that is transmitted from the server device;
reproducing, in the client device, the received second moving image data;
inputting the contents of edition processing for the second moving image data being reproduced;
transmitting, from the client device, edition processing information that indicates the input contents of edition processing;
receiving, by the server device, the edition processing information which is transmitted from the client device and indicates the contents of edition processing with respect to the second moving image data; and
editing, in the server device, the first moving image data based on the received edition processing information.

16. A program performing data processing in a medical image processing system comprising a server device processing medical image information and a client device connected to the server device through a network, the program realizing:
a function of storing first moving image data that is moving image data of a medical image and second moving image data that is generated by reducing a data amount of the first moving image data;
a function of transmitting the second moving image data to a client device according to a request from the client device;
a function of receiving edition processing information which is transmitted from the client device and indicates the contents of edition processing with respect to the second moving image data; and
a function of editing the first moving image data based on the received edition processing information.

17. A program performing data processing in a medical image processing system comprising a server device processing medical image information and a client device connected to the server device through a network, the program realizing:
a function of transmitting transmission request information for requesting transmission of second moving image data to a server device;
a function of receiving the second moving image data that is transmitted from the server device after transmission of the transmission request information;
a function of reproducing the second moving image data that is received by the client side reception unit;
a function of inputting the contents of edition processing for the second moving image data being reproduced; and
a function of transmitting the edition processing information that indicates the input contents of edition processing.
